Europäisches Patentamt

European Patent Office

Office européen des brevets

(11) Publication number: **0 322 237 B1**

# EUROPEAN PATENT SPECIFICATION

(45) Date of publication of patent specification: **23.03.94**

(51) Int. Cl.⁵: **C12N 15/01**, A61K 39/112, //A61K39/10,A61K39/102, A61K39/02,A61K39/09, A61K39/095,A61K39/04

(21) Application number: **88312203.8**

(22) Date of filing: **22.12.88**

(54) Vaccines.

(30) Priority: **23.12.87 GB 8730037**

(43) Date of publication of application:
**28.06.89 Bulletin 89/26**

(45) Publication of the grant of the patent:
**23.03.94 Bulletin 94/12**

(84) Designated Contracting States:
**AT BE CH DE ES FR GB GR IT LI LU NL SE**

(56) References cited:
**EP-A- 0 184 086**
**WO-A-80/02504**

**CHEMICAL ABSTRACTS, vol. 107, no. 7, 17th August 1987, page 194, abstract no.53112x, Columbus, Ohio, US; G. DOUGAN et al.: "Isolation of stable aroA mutants**

**of Salmonella typhi Ty2: properties and preliminary characterization in mice",& MGG, MOL. GEN. GENET. 1987, 207(2-3), 402-5**

(73) Proprietor: **THE WELLCOME FOUNDATION LIMITED**
**Unicorn House**
**160 Euston Road**
**London NW1 2BP(GB)**

(72) Inventor: **Dougan, Gordon**
**Langley Court**
**Beckenham Kent BR3 3BS(GB)**
Inventor: **Chatfield, Steven Neville**
**Langley Court**
**Beckenham Kent BR3 3BS(GB)**
Inventor: **Hormaeche, Carlos Estenio, Dr.**
**The University of Cambridge,Tennis Court Rd.**
**Cambridge CB2 1OP(GB)**

(74) Representative: **Stott, Michael John et al**
**The Wellcome Foundation Limited**
**Group Patents & Agreements**
**Langley Court**
**Beckenham Kent BR3 3BS (GB)**

CHEMICAL ABSTRACTS, vol. 106, no. 25, 22nd June 1987, page 497, abstract no.212119c, Columbus, Ohio, US; D.J. MASKELL et al.: "Salmonella typhimurium aroAmutants as carriers of the Escherichia coli heat labile enterotoxin B subunitto the murine secretory and systemic immune systems", & MICROB.PATHOG. 1987, 2(3), 211-21

BRIT. J. EXPER. PATH., vol. 32, 1951, pages 85-96; G.A. BACON et al.: "Theeffects of biochemical mutation on the virulence of bacterium typhosum: theloss of virulence of certain mutants"

BIOLOGICAL ABSTRACTS, abstract no. 87048348; G. DOUGAN et al.: "Construction and characterization of vaccine strains of Salmonella harboring mutations intwo different aro genes", & JOURNAL OF INFECTIOUS DISEASES(US) 1988. vol 158, no. 6, p. 1329-1335

VACCINE, vol. 6, no. 2, April 1988, pages 141-145, Butterworth& Co. (Publishers) Ltd, Guildford, Surrey, GB; B.A.D.

STOCKER: "Auxotrophic Salmonella typhi as live vaccine"

**Description**

The present invention relates to oral vaccines based on live genetically attenuated microorganisms and to the micro-organisms themselves. In particular the invention is directed towards attenuated strains of Salmonella.

In 1950 Bacon et al, (Br. J. Exp. Path. 31: 714-724) demonstrated that certain auxotrophic mutants of S. typhi were attenuated in mice when compared with the parental strain. Certain of these strains included mutations in the aromatic and purine biosynthetic pathway. It is also known that other mutations, such as thy A, attenuate bacteria.

In 1981 Hosieth and Stocker (Nature 241: 238-39) reported the construction of an S. typhimurium aro A mutant. The aro A mutation was constructed using transposon Tn 10 mutagenesis to construct S. typhimurium strains carrying non-reverting lesions in the aro A gene. This gene encodes the enzyme 5-enolpyruvylshikimate-3-phosphate synthase, a key enzyme in the organism's aromatic bio-synthetic pathway, which is absent in mammals. Aro A mutants are therefore dependent on exogenous aromatic compounds, including the aromatic amino acids, p-amino benzoic acid and 2,4, dihydroxybenzoate for growth. It was shown in in-bred mice that S. typhimurium aro A mutants are attenuated and were found to be effective live vaccines in mice against murine salmonellosis when delivered orally or parenterally.

If a microorganism is to be used in a live form in a vaccine preparation, safety reasons dictate that the microorganism be attenuated with at least two mutations, preferably in separate parts of the genome. It is clearly important that such a microorganism does not revert back to the virulent parent. The probability of this happening with a single mutation is considered to be small. However, the risk of reversion occurring in a strain harbouring mutations in two discrete genes, located in different places in the genome, is insignificant. A double mutant of this sort is thus considered to be a much safer candidate for use in a vaccine.

In EP-A-184086 (The Board of Trustees of the Leland Stanford Junior University; Inventor: Bruce Stocker) there is described the construction of a non-reverting strain of S. typhi which harbours aro A and pur A non-reverting mutations. Non-reverting mutations are those mutations which cannot be repaired in a single step. Genetic mutations of this sort include inversions and deletions of a DNA sequence which makes up part of a gene.

In our experiments we have shown that intravenous administration of non-reverting aro A pur A mutants of S. typhimurium performed poorly in protecting BALB/c mice against intravenous challenge. These mutants were also shown to be ineffective in protecting BALB/c mice when administered by the oral route. (O'Callaghan et al, 1988 Infect. Immun. 56, 419-423)

The aro A and pur A mutations can be prepared using transposons. These are DNA sequences of between 750 base pairs to many thousands of nucleotide pairs which can integrate their genetic material into different positions within the bacterial chromosome. Some transposons encode a gene which will confer antibiotic resistance on the organism containing the transposon. When the insertion occurs within a locus, the continuity of the gene is often interrupted and this results in the loss of gene function. At a frequency of about $10^{-8}$/cells/generation transposons are precisely deleted from the gene. This restores gene function; more frequently however, imprecise excision occurs. This does not restore gene function and often leads to a non-reverting mutation.

Some of the work carried out in support of this invention is centered on S. typhi, the cause of human typhoid. S. typhi is essentially a human pathogen and thus is not suitable for most animal experimental work. Animal studies are carried out using a mouse model and the organism S.typhimurium, a closely related organism which causes a "typhoid-like" disease in mice and cattle. A description of this mouse model can be found in Collins, 1974, Bacteriol Rev. 38, 371.

For a microorganism to be considered for use in a vaccine, it must exhibit the following properties:-
I) Sufficiently attenuated such that it substantially fails to cause the infection associated with the unattenuated microorganism;
II) substantially incapable of reversion to virulence,
III) capable of inducing immunity in an animal inoculated with the organism and thus providing protection against subsequent challenge with the virulent strain.

It is believed that the live microorganisms described in the prior art for use as vaccines have failed to fulfil all the necessary criteria noted above. Nevertheless the desirability to develop a live vaccine which avoids the short comings of the prior art remains, since it has been shown (Collins, Bacteriol Rev. 1974) that generally live bacteria have greater immunizing efficacy than killed bacteria. The present inventors have shown that introduction of a non-reverting mutation into each of two discrete aro genes in the aromatic biosynthetic pathway of a bacterium provide a suitable organism for use in a live vaccine preparation.

3

Thus according to a first aspect of the invention there is provided a attenuated bacterium harbouring a non-reverting mutation in each of two discrete aro genes of the aromatic biosynthetic pathway. In a second aspect the invention provides a method of producing an attenuated bacterium, comprising the introduction of a second non-reverting mutation into a second aro gene in a bacterium containing a first non-reverting mutation in a first aro gene.

There are at least ten genes involved in the synthesis of chorismate, the branch point compound in the aromatic amino acid biosynthetic pathway. Several of these map at widely differing locations on the bacterial genome. Aro genes include aro A (5-enolpyruvylshikimate-3-phosphate synthase), aro C - (chorismate synthase), aro D (3-dihydroquinate dehydratase) and aro E (shikimate dehydrogenase).

Thus in preferred embodiments of the present invention one of the mutations occurs in the aro A, aro C, aro D or aro E genes. In three embodiments, the invention provides aro A aro E mutant bacteria, aro A aro C mutant bacteria and aro A aro D mutant bacteria although other double aro mutants are within the scope of the present invention. An attenuated bacterium of the invention typically may have one of the non-reverting mutations occuring in the aro A gene. A second non-reverting mutation may then typically occur in one of the aro C and aro E genes.

In particular this work can be extended to a whole range of bacterial pathogens (especially invasive bacterial pathogens which invade and grow within eucaryotic cells or colonise muscosal surfaces). Examples of these include members of the genera Salmonella, Bordetella and Haemophilus, Other examples are members of the genera Neisseria, Mycobacteria, Leptospira and Streptococcus. Particular examples are S.typhi, the cause of human typhoid; S.typhimurium the cause of salmonellosis in several animal species; S.enteritidis a cause of food poisoning in humans; S.cholerasuis, the cause of salmonellosis in pigs; Bordetella pertussis the cause of whooping cough; Haemophilus influenzae, a cause of meningitis; Mycobacterium tuberculosis, the cause of tuberculosis and Neisseria gonorrhoeae the cause of gonorrhoea, Yersinia pestas, the cause of bubonic plague.

In a preferred embodiment of the invention there is provided a S. typhi strain Ty 2 harbouring either aro A aro C or aro A aro E non-reverting mutations or aro A aro D non-reverting mutations
The construction of S. typhi Ty 2 aro A is documented in MGG 207, 402 (Dougan et al). Non-reverting mutations were generated by transducing an LT2 aro A:: Tn 10 marker into S. typhi Ty 2 strain. Tn 10 transposon carries a gene encoding for tetracycline resistance. Transductants are selected that are tetracycline resistant by growing colonies on an appropriate medium. Further selection is undertaken by screening for those bacteria which have lost the tetracycline resistance gene and which are also aromatic dependent.

An alternative method for introducing a deletion into the S.typhi aro A gene (or other S.typhi aro genes) involves transposon mutagenesis of a cloned S.typhi aro A gene, recombination of the mutated gene into the S.typhi chromosome replacing the wild-type gene with the mutant and selection for imprecise excision of the transposon. This method eliminates the introduction of non S.typhi DNA into the vaccine strain.

In principle there are several ways of introducing the second mutation into the second gene. One method involves transposon mutagenesis i.e. the insertion of a transposable element into the second gene and then relying on its imprecise excision by the bacterium in the same manner as decribed above for constructing the first mutation. The introduction of a mutation into a second aro gene produces a double aro mutant. This is phenotypically indistinguishable from a single aro mutant. Thus to complement the first mutated aro gene, a cloned non-mutated copy of one of the genes is introduced on a plasmid into the bacterium and the bacterium checked for aromatic compound dependence using the appropriate selection medium.

Another method involves cloning the second gene into a vector eg a plasmid or cosmid, and then incorporating a selectable marker gene into the cloned second gene at the same time inactivating that gene. A plasmid carrying the inactivated gene and a different selectable marker can be introduced into the bacterium by known techniques. It is then possible by suitable selection to identify a mutant wherein the inactivated gene has recombined into the chromosome of the bacterium and the bacterium's own copy of the gene has been lost. In particular, the vector used is one which is unstable in the bacterium and will be spontaneously lost. The mutated gene on the plasmid and the bacterium's own copy of the gene on its chromosome maybe exchanged by a genetic cross-over event. Additional methods eliminate the introduction of foreign DNA into vaccine strains at the site of mutations.

The aro A aro C and aro A aro E and aro A aro D mutants of the present invention are sufficiently attenuated to be substantially safe for use in vaccines, and are sufficiently immunogenic to raise an immune response in a patient which will afford the patient protection on subsequent challenge with the virulent strain.

4

An attenuated bacterium of the invention may be capable of expressing a heterologous antigen. The strains of the present invention may be genetically engineered so as to express antigens from one or more different pathogens. Such pathogens, maybe viral, bacterial, protozoal or of higher parasitic organisms. The pathogens may infect both humans and other mammals, but maybe species selective, or even species specific. Such strains could then form the basis of a bi or multivalent vaccine. Examples of useful antigens include E. coli heat labile toxin B subunit (LT-B) E. coli K88 antigens, FMDV (Foot and Mouth) peptides, Influenza viral proteins, 69Kd protein from B.pertussis. Other antigens which could be usefully expressed would be those from Chlamydia, flukes, mycoplasma, roundworms, tapeworms, rabies virus and rotavirus.

These antigens may be produced by the introduction of the gene or genes encoding them into expression cassettes. Expression cassettes will include DNA sequences, in addition to that coding for the structural gene, which will encode for transcriptional and translational initiation and termination regions. The expression cassette may also include regulatory regions. Such expression cassettes are well known in the art and it is well within the skill of the skilled man to construct them. The expression cassette may be a construct or a naturally occuring plasmid. An example of a genetically engineered attenuated Salmonella which expresses a foreign antigen can be found in EP-A-0 127 153 (SSVI/Wellcome). The expression cassette may also be engineered to allow the incorporation of the heterologous gene into the bacterial chromosome.

A further bivalent vaccine comprising an attenuated Salmonella typhi, capable of expressing the E.coli heat-labile enterotoxin subunit B was disclosed by Clements et al (Infection and Immunity, 46, No.2., Nov 1984, p564-569). Ty21a has been used to express other antigens such as the Shigella sonnei form I antigen (Formal et al, Infection and Immunity 34 746-750).

According to a third aspect of the invention there is provided an attenuated bacterium, as herein described, transformed with an expression cassette encoding an antigen of a pathogen, wherein in use said antigen is expressed by said attenuated bacterium.

According to a fourth aspect of the invention we provide a pharmaceutical composition which comprises an attenuated bacterium as herein described in admixture with a pharmaceutically acceptable carrier. Preferably the pharmaceutical composition is a vaccine composition.

The vaccine is advantageously presented in a lyophilised form, for example in a capsular form, for oral administration to a patient. Such capsules may be provided with an enteric coating comprising for example Eudragate "S", Eudragate "L", Cellulose acetate, cellulose phthalate or hydroxypropylmethyl cellulose. These capsules may be used as such, or alternatively, the lyophilised material may be reconstituted prior to administration, e.g. as a suspension. Reconstitution is advantageously effected in a buffer at a suitable pH to ensure the viability of the organisms. In order to protect the attenuated bacteria and the vaccine from gastric acidity, a sodium bicarbonate preparation is advantageously administered before each administration of the vaccine. Alternatively the vaccine may be prepared for parenteral administration, intranasal administration or intramammory.

A method for the prophylactic treatment of a bacterial infection which comprises administering to a patient an effective dose of the an attenuated bacterium of the invention. The dosage employed will be dependent on various clinical factors, including the size and weight of the patient, the type of vaccine formulated. However, for attenuated S. typhi a dosage comprising the administration of $10^9$ to $10^{11}$ S. typhi organisms per dose is generally convenient for a 70kg adult human patient.

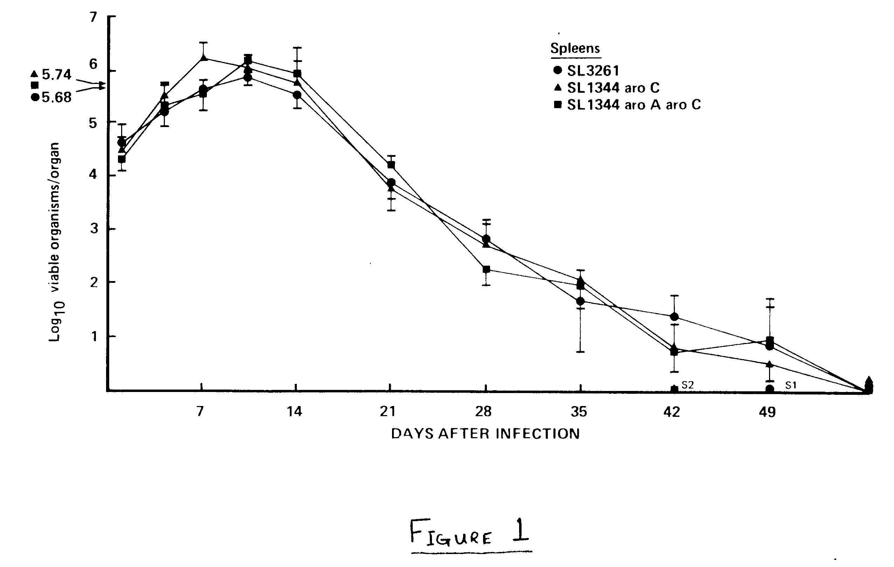

In the following, Examples are provided of experimental details in accordance with the present invention. It will be understood that these Examples are not intended to limit the invention in any way. Figure 1, which is referred to in the Examples, illustrates the persistence of S.typhimusium SL3261, SL1344 aro C and SL1344 aro A aro C in the spleens of mice. $Log_{10}$ viable organisms/organ is plotted against days after infection.

Construction of S. typhi aro A, aro E and aro A, aro C and aro A aro D vaccine strains

All strains were constructed using as a starter strain S. typhi Ty2 aro A described in detail previously and the subject of Dougan et al Mol. Gen. Genet. (1987) 207: 402-405.

Example 1.

S. typhi Ty2 are A aro E

Strain S. typhimurium LT2 are E::Tn10 was obtained from the Salmonella Stock Centre in Calgary. It was originally isolated by J. Roth. Phage P22 was grown on LT2 aro E::Tn10 to prepare a lysate. The P22

5

phage lysate was used to transduce S. typhi Ty2 aro A selecting for tetracycline resistance. At this point a plasmid encoding a cloned aroA gene from S. typhimurium C5 to complement the aro A mutation was introduced into the S. typhi aro A strain. S. typhi aro A, aro E::Tn10 carrying the cloned aro A gene was phenotypically dependent on the aromatic compounds (normally required by aro mutants). The Tn 10 element was removed from the aro E gene by selecting tetracycline sensitive variants on Bochner medium, a technique used previously (Bochner et al, J. Bacteriol, 143, 929-933). S. typhi aro A aro E mutants harbouring the cloned aro A gene were checked for aromatic dependence using minimal medium. Aromatic dependent colonies were selected and checked extensively for aro E reversion, by plating $10^{11}$ organism on minimal medium lacking aromatic compounds and incubating the medium at 37°C and checking over five days for revertant colonies. Colonies which were stably aro E despite exhaustive screening were propagated to select variants which had spontaneously lost the cloned aro A gene. One S. typhi aro A aro E mutant was selected. This has been deposited at the National Collection of Type Cultures, 61 Colindale Avenue, London NW9 5HT under accession No. 12164, dated 25th November 1987, in accordance with the terms of the Budapest Treaty.

Example 2: S. typhi Ty2 aro A aro C

Strain S. typhi Ty2 aro A was used as a starter strain. The aro C gene of S. typhi Ty2 was cloned using cosmids to complement E. coli aro C using the methods of Hohn and Collins (Gene 11: 291-298 (1978)). The aro C cosmid was subjected to transposon Tn 5 mutagenesis and subcloned to locate a small DNA fragment encoding the cloned aro C gene. The cloned aro C gene was inactivated by cloning a Mercury metal resistance gene into the coding region for aro C. A plasmid carrying the inactivated aro C was introduced into S. typhi aro A. This plasmid also contains a gene for ampicillin resistance. By selecting for mercury resistance and ampicillin sensitivity it was possible to identify a mutant wherein an inactivated aro C had recombined into the S. typhi chromosome to generate an S. typhi aro A aro C mutant.

An alternative construct has been made which involved the use of a Kanamycin resistance gene (Km-R), in place of the mercury metal resistance gene.

The S. typhi Ty2 aro A aro C Km-R has been deposited at the National Collection of Type Cultures, 61 Colindale Avenue, London NW9 5HT under accession No. 12165, dated 25th November 1987, in accordance with the terms of the Budapest Treaty.

Example 3: Construction of a Salmonella typhi aroA aroD double mutant

S.typhi aro A was used as the starter strain. Construction of the S.typhi aro A aro D was achieved by transducing the strain with a P22 phage lysate prepared using donor strain LT2 aro D553::Tn10 and selecting for tetracyline resistance. One isolate was purified and used to prepare tetracyline sensitive derivatives by selection on Bochner medium. Several of these were purified and transformed with plasmid pAB51 (aroA+) to complement the aro A deletion. One of the tetracyline sensitive isolates that was stably aromatic compound dependent when harbouring this plasmid was designated S.typhi Ty2 aro A aro D.

All the S.typhi strains constructed harbouring mutations in different aro genes still produced Vi antigen, were '0' inagglutinable, 09 agglutinable following boiling and were of flagella type Hd. One such aro A aro D strain has been deposited at the National Collection of Type Cultures, 61 Colindale Avenue, London, under no. NCTC 12209, dated 15th December 1988, in accordance with the terms of the Budapest Treaty.

Example 4: Construction of double aro mutants in S.typhimurium, S.dublin, and S.cholerasuis.

An aro A deletion was introduced into S.typhimurium SL1344, S.dublin, S.cholerasuis using the method of McFarland and Stocker. A phage lysate prepared from strain TT472 was used to transduce all the Salmonella strains, selecting for tetracycline-resistant colonies. Strain TT472 carries Tn 10 inserted within ser C which is upstream of and within the same operon as aro A. Tetracycline-resistant transductants were aromatic compound, serine and pyridoxine dependent. A second P22 lysate was prepared, grown on SL5254, which has a known deletion within aro A. This was used to transduce the tetracycline resistant strains which were ser C::Tn10 and transductants were selected on minimal medium lacking serine and pyridoxine but containing aromatic compounds. Colonies growing on minimal medium with added aromatic compounds but in the absence of serine and pyridoxine were tetracycline-sensitive and aromatic compound dependent.

Example 4a: Construction of S.typhimurium aro A aro C.

S.typhimurium aro A aro C was constructed by first moving a stable aro C mutation from the avirulent S.typhimurium strain SA2018 into the mouse-virulent S.typhimurium strain SL1344 using the following series of transductions. A P22-transducing lysate prepared using S.typhimurium strain SGSC592 zei608::Tn10 was used to transduce Tn 10 into strain SA2018. The Tn 10 in strain SGSC592 is 40% linked to the wild type aro C gene, whereas strain SA2018 harbours a stable aro C mutation. Tetracycline resistant transductants were picked onto minimal medium and several colonies were found to be aromatic dependent. One of these isolates was purified and used to prepare a second P22 phage lysate. This lysate was used to transduce S.typhimurium SL1344 and again several tetracycline-resistant, aromatic compound-dependent transductants were identified. One isolate was used to prepare tetracycline sensitive derivatives by selection on Bochner medium. One tetracycline-sensitive aromatic compound dependent isolate was purified and an aro A deletion introduced into it using the method of McFarland and Stocker as described previously. To confirm that this isolate was aro A aro C it was transformed with either plasmid pAB51 (aro A$^+$) or pTMC12H (aro C$^+$) and was found to be aromatic compound dependent when it harboured either of these plasmids.

Example 4b: Construction of S.typhimurium aro A aro D

S.typhimurium aro A aro D was constructed by introducing an aro D deletion into the SL1344 aro A strain. This was achieved by transducing the strain with a P22 phage lysate prepared using donor strain LT2 aro D553::Tn10 and selecting for tetracycline resistance. One isolate was purified and used to prepare tetracycline-sensitive derivatives by selection on Bochner medium. Several of these were purified and transformed with plasmid pAB51 (aro A$^+$) to complement the aro A deletion. One of the tetracycline-sensitive isolates that was stably aromatic compound dependent when harbouring this plasmid was designated SL1344 aro A aro D.

Example 4c: Construction of S. typhimurium aro A aro E

S.typhimurium aro A aro E was constructed by introducing an aro E deletion into the SL1344 aro A strain. This was achieved by transducing the strain with a P22 phage lysate prepared using donor strain LT2 aro E::Tn10 and selecting for tetracycline resistance. One isolate was purified and used to prepare tetracycline sensitive derivatives by selection on Bochner medium. Several of these were purified and transformed with plasmid pAB51 (aro A$^+$) to complement the aro A deletion. One of the tetracycline-sensitive isolates that was stably aromatic compound dependent when harbouring this plasmid was designated SL1344 aro A aro E.

Example 5:

S.dublin and S.cholerasuis aro A aro D derivatives were constructed in the same way as the SL1344 aro A aro D derivative as shown in Example 4b.

In vivo properties of attenuated S.typhimurium strains

Details of the S. typhimurium stains used in this work are shown in Table 1.

Infection of mice and enumeration of bacteria in murine organs

To determine the number of organisms in various organs after tnt i.v. inoculation of S.typhimurium, liver and spleens were homogenised as described in Hormaeche, CE, Immunology, 37, 311-318. Viable counts were performed on these homogenates using L-agar as growth medium and are expressed in Figure 1 as geometric means ± two standard errors of the mean, for four mice per point.

Innately Salmonella susceptible BALB/c mice of 8-10 weeks of age were used throughout examples 6, 7 and 8. The intravenous (i.v.) LD$_{50}$ for virulent strains was obtained by injecting groups of 5 mice with serial ten fold dilutions, prepared in phosphate buffered saline pH 7.2 (PBS), of overnight L-broth cultures harvested by centrifugation and resuspended in PBS to give a concentration of $10^9$ - $10^{11}$ bacteria per ml. This was serially ten fold diluted in PBS, the top dose being 0.2 ml of the neat suspension given i.v. or orally with 8-10 mice per group. Deaths were recorded over the following four weeks and the LD$_{50}$ was

calculated using the method of Reed and Muench Am. J. Hyg. $\underline{27}$: 493-497(1983). For i.v. inoculation mice were injected with 0.2 ml of bacterial suspension into the tail vein. For oral inoculation bacteria were administered in 0.2 ml volumes to lightly ether anaesthetised mice by gavage as described previously (Microbial Pathogenesis $\underline{2}$: 211-221).

Example 6.

Attenuation of virulent S. typhimurium strains by the introduction of a stable aro mutations and other auxotrophic mutations.

S. typhimurium HWSH and SL1344 are both mouse virulent strains with an $LD_{50}$ of less than 10 organisms following i.v. challenge in BALB/c mice. Intravenous $LD_{50}$s were determined for selected auxotrophic derivatives of these strains in BALB/c mice (Table 2). All aro A and pur A mutants were well attenuated compared to the parental strains. HWSH aro A and SL3261 (SL1344 aro A) had $LD_{50}$s of log 7.4 and log 7.1 respectively in good agreement with published data. The pur A and aro A pur A derivatives were even more attenuated. The HWSH pur E strain was considerably less attenuated though the measured $LD_{50}$ was found to vary considerably between experiments. For example, sometimes mice given as few as 100 organisms died whereas others given up to $10^4$ - $10^5$ organisms survived. The aro C aro A mutant was less attenuated than the aro A pur A mutant having an $LD_{50}$ of 6.5. This figure was the same for the single aro C mutant. Orally, aromatic or purine mutants did not kill the mice even when doses as high as $10^{10}$ organisms were given.

Example 7

Persistance of S.typhimurium single and double aro mutants after i.v. inoculation of BALB/c mice.

All S.typhimurium strains exhibited a very similar pattern of persistance, with no salmonellae detectable in spleens by day 56 (Fig 1).

Attenuated strains of S.typhimurium as orally administered vaccines.

Example 8

The ability of orally administered auxotrophic S. typhimurium strains to protect BALB/c mice against an oral challenge with virulent S. typhimurium (SL1344) was treated. Mice were initially infected orally with between $10^9$ - $10^{10}$ of the auxotrophic mutants and then the immunised mice were challenged orally four weeks later with the parental virulent strain.

The results in Table 3 clearly show that neither pur A nor aro A pur A mutants induced any significant protection against oral challenge whereas the aro A, aro C and aro A aro C mutants did induce significant protection against oral challenge at both 28 and 70 days post immunisation.

Example 9: Formulation

An S. typhi organism of the present invention is preferably presented in oral tablet form.

| Ingredient | mg/tablet |
|---|---|
| **Core-tablets** | |
| 1) Freeze-dried excipient carrier containing $10^9$-$10^{10}$ Salmonella typhi. | 70.0 |
| 2) Silica dioxide (Aerosil 200) | 0.5 |
| 3) Dipac (97% sucrose) | 235.0 |
| 4) Cross-linked Povidone (Kollidon CL) | 7.0 |
| 5) Microcrystalline Cellulose (Avicel pH 102) | 35.0 |
| 6. Magnesium Stearate | 2.5 |
| | 350.0 |

9

Coating

7)  Opadry Enteric, OY-P-7156

(Polyvinyl acetate phthalate

+ Diethylphthate)                                            35.0

─────

385.0

─────

A carrier containing 5% sucrose 1% sodium glutamate and 1% bacto casitone in an aqueous solvent is prepared. S. typhi organism are suspended in this carrier and then subjected to freeze-drying.

The freeze-dried material is blended with Aerosil 200 and the blended mixture is sifted through a screen. The sifted powder is mixed with Dipac, Kollidan CL, Aricel pH 102 and Magnesium Stearate in a blender. This blend is compressed into tablets for subsequent enteric coatings.

The skilled man will appreciate that many of the ingredients in this formulation could be replaced by functionally equivalent pharmaceutically acceptable excipients.

Table 1

| Strains of S. typhimurium used in this study. | |
| --- | --- |
| Strain | Auxotrophy |
| SL1344 | his |
| SL3261 | aro A his |
| SL1344 pur A | pur A his |
| SL3261 pur A | pur A aro A his |
| SL1344 | aro C |
| SL1344 | aro A |
| SL1344 | aro A aro C |
| HWSH* | Prototroph |
| HWSH aro A | aro A |
| HWSH pur A | pur A |
| HWSH aro A pur A | aro A pur A |
| HWSH pur E | pur E |
| C5 | Prototroph |

* A mouse-virulent, calf-virulent strain isolated from a calf dying of Salmonellosis.

Table 2

| Log LD$_{50}$ values obtained by infecting BALB/c mice i.v. with various auxotrophic derivatives of two highly virulent S. typhimurium strains[a]. | | | |
|---|---|---|---|
| | Log LD$_{50}$ | | Log LD$_{50}$ |
| SL1344 | <1 | HWSH | <1 |
| SL3261 | 6.9 | HWSH aro A | 7.4 |
| SL1344 pur A | 6.7 | HWSH pur A | 8.6 |
| SL3261 pur A | 8.7 | HWSH aro A pur A | 8.9 |
| SL1344 aro C | 6.7 | HWSH pur E | 3.8 |
| SL1344 aro C aro A | 6.5 | | |
| SL1344 aro A | 6.75 | | |

[a] All LD$_{50}$s were calculated after 28 days, except for HWSH pur E, calculated after 56 days.

Table 3

| Immunising strain | Challenge Day 28 | Challenge Day 70 |
|---|---|---|
| SL1344 pur A | 6.7 | ND |
| SL3261 pur A | 5.6 | ND |
| Unimmunised | 6.2 | ND |
| SL3261 | >10.1 | 9.1 |
| SL1344 aro C | >10.1 | >9.9 |
| SL1344 aro A aro C | >10.1 | >9.9 |
| Unimmunised | 7.4 | 6.7 |
| Note: SL 3261 is aro A | | |

**Claims**

**Claims for the following Contracting States : AT, BE, CH, DE, FR, GB, IT, LI, LU, NL, SE**

1. An attenuated bacterium harbouring a non-reverting mutation in each of two discrete aro genes of the aromatic biosynthetic pathway.

2. An attenuated bacterium as claimed in claim 1 wherein one of the non-reverting mutations occurs in the aro A gene.

3. An attenuated bacterium as claimed in claim 2 wherein a second non-reverting mutation occurs in one of the aro C and aro E genes.

4. An attenuated bacterium as claimed in any one of the preceding claims wherein the microorganism is selected from the genera Salmonella, Bordetella and Haemophilus.

5. An attenuated bacterium as claimed in claim 4 wherein the bacterium is S.typhi.

6. An attenuated bacterium S.typhi strain Ty2 harbouring either aro A aro C or aro A aro E non-reverting mutations.

7. An attenuated bacterium according to claim 5 which is a strain of S.typhi as deposited under the national Collection of Type Cultures' Accession No. 12164 or 12165.

8. An attenuated bacterium as claimed in any one of the preceding claims capable of expressing a heterologous antigen.

**9.** An attenuated bacterium as claimed in claim 8 containing an expression cassette having a DNA sequence encoding an antigen of a pathogen.

**10.** An attenuated bacterium as claimed in any one of the preceding claims for use in the prophylactic treatment of a bacterial infection.

**11.** An attenuated S.typhi organism as claimed in any one of claims 5 to 7 for use in the prophylactic treatment of typhoid in humans.

**12.** A vaccine comprising an attenuated bacterium as defined in any one of the preceding claims in admixture with a pharmaceutically acceptable excipient.

**13.** A method of producing an attenuated bacterium comprising the introduction of a second non-reverting mutation into a second aro gene in a bacterium containing a first non-reverting mutation in a first aro gene.

**14.** A method as claimed in claim 13 wherein the second mutation is introduced by transposon mutagenesis.

**15.** A method as claimed in claim 13, comprising cloning the second aro gene into a vector, incorporating a selectable marker gene into the cloned gene thereby to inactivate the cloned gene, introducing a plasmid carrying the inactivated gene and a different selectable marker gene into the bacterium and selecting a mutant of the said bacterium wherein the inactivated gene has recombined into the chromosome of the bacterium.

**Claims for the following Contracting States : ES, GR**

**1.** A method of producing an attenuated bacterium comprising the introduction of a second non-reverting mutation into a second aro gene in a bacterium containing a first non-reverting mutation in a first aro gene.

**2.** A method as claimed in claim 1 wherein the second mutation is introduced by transposon mutagenesis.

**3.** A method as claimed in claim 1, comprising cloning the second aro gene into a vector, incorporating a selectable marker gene into the cloned gene thereby to inactivate the cloned gene, introducing a plasmid carrying the inactivated gene and a different selectable mark gene into the bacterium and selecting a mutant of the said bacterium wherein the inactivated gene has recombined into the chromosome of the bacterium.

**4.** A method as claimed in any one of the preceding claims, wherein one of the aro genes is the aro A gene.

**5.** A method as claimed in claim 4 wherein the second aro gene is the aro C or aro E gene.

**6.** A method as claimed in any one of the preceding claims wherein the bacterium is selected from the genera Salmonella, Bordetella and Haemophilus.

**7.** A method as claimed in claim 6 wherein the bacterium is S.typhi.

**8.** A method as claimed in claim 7 wherein the bacterium is S.typhi strain Ty2.

**9.** A method according to any one of the preceding claims, in which the attenuated bacterium thus produced harbouring a non-reverting mutation in each of two discrete aro genes in its aromatic biosynthetic pathway is provided with an expression cassette having a DNA sequence encoding an antigen of a pathogen.

**10.** A method according to any one of the preceding claims, in which the attenuated bacterium thus produced harbouring a non-reverting mutation in each of two discrete aro genes of its aromatic

EP 0 322 237 B1

biosynthetic pathway is admixed with a pharmaceutically acceptable carrier.

11. A method of producing a vaccine comprising admixing an attenuated bacterium harbouring a non-reverting mutation in each of two discrete aro genes in its aromatic biosynthetic pathway with a pharmaceutically acceptable carrier.

12. A method according to claim 11, wherein one of the aro genes is the aro A gene.

13. A method according to claim 12, wherein the second aro gene is the aro C or aro E gene.

14. A method according to any one of claims 11 to 13, wherein the bacterium is selected from the genera Salmonella, Bordetella and Haemophilus.

15. A method according to claim 14, wherein the bacterium is S.typhi.

**Patentansprüche**
**Patentansprüche für folgende Vertragsstaaten : AT, BE, CH, DE, FR, GB, IT, LI, LU, NL, SE**

1. Attenuiertes Bakterium, das eine nicht-revertierende Mutation in jedem von zwei separaten aro-Genen des aromatischen anabolen Stoffwechselswegs beherbergt.

2. Attenuiertes Bakterium nach Anspruch 1, bei dem eine der nicht-revertierenden Mutationen in dem aro A-Gen auftritt.

3. Attenuiertes Bakterium nach Anspruch 2, bei dem eine zweite nicht-revertierende Mutation in einem der Gene: aro C-Gen und aro E-Gen auftritt.

4. Attenuiertes Bakterium nach einem der vorangehenden Ansprüche, wobei der Mikroorganismus unter den Gattungen Salmonella, Bordetella und Haemophilus ausgewahlt ist.

5. Attenuiertes Bakterium nach Anspruch 4, wobei das Bakterium S.typhi ist.

6. Attenuiertes Bakterium S.typhi, Stamm Ty2, das entweder aro A aro C- oder aro A aro E- nichtrevertierende Mutationen beherbergt.

7. Attenuiertes Bakterium nach Anspruch 5, welches ein Stamm von S.typhi ist, wie er in der National Collection of Type Cultures unter der Zugriffsnummer 12 164 oder 12 165 hinterlegt ist.

8. Attenuiertes Bakterium nach einem der vorangehenden Ansprüche, das fähig ist, ein heterologes Antigen zu exprimieren.

9. Attenuiertes Bakterium nach Anspruch 8, das eine Expressionskassette mit einer DNA-Sequenz enthält, die ein Antigen eines Krankheitserregers kodiert.

10. Attenuiertes Bakterium nach einem der vorangehenden Ansprüche zur Verwendung in der prohpylaktischen Behandlung einer bakteriellen Infektion.

11. Attenuierter S.typhi-Organismus nach einem der Ansprüche 5 bis 7 zur Verwendung in der prophylaktischen Behandlung von Thyphus bei Menschen.

12. Vakzin umfassend ein attenuiertes Bakterium nach einem der vorangehenden Ansprüche im Gemisch mit einer pharmazeutisch akzeptablen Arzneimittelträgersubstanz.

13. Verfahren zur Herstellung eines attenuierten Bakteriums, welches die Einführung einer zweiten nicht-revertierenden Mutation in ein zweites aro-Gen in einem Bakterium, das eine erste nicht-revertierende Mutation in einem ersten aro-Gen enthält, umfaßt.

13

**14.** Verfahren nach Anspruch 13, bei dem die zweite Mutation durch Transposon-Mutagenese eingeführt wird.

**15.** Verfahren nach Anspruch 13, umfassend Klonierung des zweiten aro-Gens in einen Vektor; Einbauen eines selektierbaren Markergens in das geklonte Gen, um dadurch das geklonte Gen zu inaktivieren: Einführen eines Plasmids, das das inaktivierte Gen und ein anderes selektierbares Markergen trägt, in das Bakterium sowie Selektieren eines Mutanten dieses Bakteriums, in dem eine Rekombination des inaktivierten Gen in das Chromosom des Bakteriums erfolgt ist.

**Patentansprüche für folgende Vertragsstaaten : ES, GR**

**1.** Verfahren zur Herstellung eines attenuierten Bakteriums, welches die Einführung einer zweiten nicht-revertierenden Mutation in ein zweites aro-Gen in einem Bakterium, das eine erste nicht-revertierende Mutation in einem ersten aro-Gen enthält, umfaßt.

**2.** Verfahren nach Anspruch 1, bei dem die zweite Mutation durch Transposon-Mutagenese eingeführt wird.

**3.** Verfahren nach Anspruch 1, umfassend Klonierung des zweiten aro-Gens in einen Vektor; Einbauen eines selektierbaren Markergens in das geklonte Gen, um dadurch das geklonte Gen zu inaktivieren, Einführen eines Plasmids, das das inaktivierte Gen und ein anderes selektierbares Markergen trägt, in das Bakterium sowie Selektieren einer Mutanten dieses Bakteriums, in der eine Rekombination des inaktivierten Gen in das Chromosom des Bakteriums erfolgt ist.

**4.** Verfahren nach einem der vorangehenden Ansprüche, bei dem eine der aro-Gene das aro A-Gen ist.

**5.** Verfahren nach Anspruch 4, bei dem das aro-Gen das aro C-Gen oder das aro E-Gen ist.

**6.** Verfahren nach einem der vorangehenden Ansprüche, bei dem das Bakterium unter den Gattungen Salmonella, Bordetella und Haemophilus ausgewählt wird.

**7.** Verfahren nach Anspruch 6, bei dem das Bakterium S.typhi ist.

**8.** Verfahren nach Anspruch 7, bei dem das Bakterium S.typhi, Stamm Ty2 ist.

**9.** Verfahren nach Anspruch 6, bei dem das so hergestellte attenuierte Bakterium, das in jedem von zwei separaten aro-Genen in seinem aromatischen anabolen Stoffwechselweg eine nicht-revertierende Mutation beherbergt, mit einer Expressionskassette, die eine DNA-Sequenz enthält, welche ein Antigen eines Krankheitserregers kodiert, bereitgestellt wird.

**10.** Verfahren nach Anspruch 6, bei dem das so hergestellte attenuierte Bakterium, das in jedem von zwei separaten aro-Genen seines aromatischen anabolen Stoffwechselwegs eine nicht-revertierende Mutation beherbergt, einer pharmazeutisch akzeptablen Arzneimittelträgersubstanz zugemischt wird.

**11.** Verfahren zur Herstellung eines Vakzins, welches das Zumischen eines attenuierten Bakteriums, das in jedem von zwei separaten aro-Genen seines aromatischen anabolen Stoffwechselwegs eine nicht-revertierende Mutation beherbergt, zu einer pharmazeutisch akzeptablen Arzneimittelträgersubstanz, umfaßt.

**12.** Verfahren nach Anspruch 12, bei dem eines der aro-Gene das aro A-Gen ist.

**13.** Verfahren nach Anspruch 12, bei dem eines der aro-Gene das aro C- oder das aro E-Gen ist.

**14.** Verfahren nach einem der Ansprüche 11 bis 13, bei dem das Bakterium unter den Gattungen Salmonella, Bordetella und Haemophilus ausgewählt wird.

**15.** Verfahren nach Anspruch 14, bei dem das Bakterium S.typhi ist.

EP 0 322 237 B1

**Revendications**
**Revendications pour les Etats contractants suivants : AT, BE, CH, DE, FR, GB, IT, LI, LU, NL, SE**

1. Bactérie atténuée portant une mutation non réverse dans chacun de deux gènes aro discrets de la voie biosynthétique aromatique.

2. Bactérie atténuée suivant la revendication 1, dans laquelle l'une des mutations non réverses se situe dans le gène aro A.

3. Bactérie atténuée suivant la revendication 2, dans laquelle une deuxième mutation non réverse se situe dans l'un des gènes aro C et aro E.

4. Bactérie atténuée suivant l'une quelconque des revendications précédentes, dans laquelle le microorganisme est choisi parmi les genres Salmonella, Bordetella et Haemophilus.

5. Bactérie atténuée suivant la revendication 4, dans laquelle la bactérie est S. typhi.

6. Bactérie atténuée de la souche S. typhi Ty2 portant les mutations non réverses soit aro A aro C, soit aro A aro E.

7. Bactérie atténuée suivant la revendication 5, qui est une souche de S. typhi comme déposé sous le numéro d'entrée national du Collection of Type Cultures n° 12164 ou 12165.

8. Bactérie atténuée suivant l'une quelconque des revendications précédentes capable d'exprimer un antigène hétérologue.

9. Bactérie atténuée suivant la revendication 8, contenant une cassette d'expression ayant une séquence d'ADN codant un antigène ou un pathogène.

10. Bactérie atténuée suivant l'une quelconque des revendications précédentes, à utiliser dans le traitement prophylactique d'une infection bactérienne.

11. Organisme S. typhi atténué suivant l'une quelconque des revendications 5 à 7, à utiliser dans le traitement prophylactique de la typhoïde chez l'humain.

12. Vaccin comprenant une bactérie atténuée comme défini dans l'une quelconque des revendications précédentes, en mélange avec un excipient pharmaceutiquement acceptable.

13. Procédé pour produire une bactérie atténuée comprenant l'introduction d'une deuxième mutation non réverse dans un deuxième gène aro, dans une bactérie contenant une première mutation non réverse dans un premier gène aro.

14. Procédé suivant la revendication 13, dans lequel la deuxième mutation est introduite par mutagénèse de transposon.

15. Procédé suivant la revendication 13, comprenant le clonage du deuxième gène aro dans un vecteur, incorporation d'un gène marqueur susceptible de sélection dans le gène cloné afin d'inactiver le gène cloné, introduction d'un plasmide portant le gène inactivé et un autre gène marqueur susceptible de sélection dans la bactérie et sélection d'un mutant de la bactérie où le gène inactivé a recombiné dans le chromosome de la bactérie.

**Revendications pour les Etats contractants suivants : ES, GR**

1. Procédé pour produire une bactérie atténuée comprenant l'introduction d'une deuxième mutation non réverse dans un deuxième gène aro dans une bactérie contenant une première mutation non réverse dans un premier gène aro.

15

**2.** Procédé suivant la revendication 1, dans lequel la deuxième mutation est introduite par mutagénèse de transposon.

**3.** Procédé suivant la revendication 1, comprenant le clonage du deuxième gène aro dans un vecteur, l'incorporation d'un gène marqueur susceptible de sélection dans le gène cloné pour inactiver le gène cloné, l'introduction d'un plasmide portant le gène inactivé et un autre gène marqueur susceptible de sélection dans la bactérie et la sélection d'un mutant de la bactérie où le gène inactivé a recombiné le chromosome de la bactérie.

**4.** Procédé suivant l'une quelconque des revendications précédentes, dans lequel un des gènes aro est le gène aro A.

**5.** Procédé suivant la revendication 4, dans lequel le deuxième gène aro est le gène aro C ou aro E.

**6.** Procédé suivant l'une quelconque des revendications précédentes, dans lequel la bactérie est choisie parmi les genres Salmonella, Bordetella et Haemophilus.

**7.** Procédé suivant la revendication 6, dans lequel la bactérie est S. typhi.

**8.** Procédé suivant la revendication 7, dans lequel la bactérie est la souche Ty2 de S. typhi.

**9.** Procédé suivant l'une quelconque des revendications précédentes, dans lequel la bactérie atténuée ainsi produite portant une mutation non réverse de chacun de deux gènes aro discrets dans sa voie biosynthétique aromatique, est munie d'une cassette d'expression ayant une séquence d'ADN codant un antigène ou un pathogène.

**10.** Procédé suivant l'une quelconque des revendications précédentes, dans lequel la bactérie atténuée ainsi produite portant une mutation non réverse dans chacun de deux gènes aro discrets de sa voie biosynthétique aromatique, est mélangée à un excipient pharmaceutiquement acceptable.

**11.** Procédé de production d'un vaccin comprenant le mélange d'une bactérie atténuée portant une mutation non réverse dans chacun de deux gènes aro discrets dans sa voie biosynthétique aromatique avec un excipient pharmaceutiquement acceptable.

**12.** Procédé suivant la revendication 11, dans lequel un des gènes aro est le gène aro A.

**13.** Procédé suivant la revendication 12, dans lequel le deuxième gène aro est le gène aro C ou aro E.

**14.** Procédé suivant l'une quelconque des revendications 11 à 13, dans lequel la bactérie est choisie parmi les genres Salmonella, Bordetella et Haemophilus.

**15.** Procédé suivant la revendication 14, dans lequel la bactérie est S. typhi.

FIGURE 1